## Europäisches Patentamt
## European Patent Office
## Office européen des brevets

(11) Veröffentlichungsnummer: **0 053 793**
**B1**

(12)
# EUROPÄISCHE PATENTSCHRIFT

(45) Veröffentlichungstag der Patentschrift:
16.05.84

(51) Int. Cl.³: **C 07 C 45/90, C 07 C 49/90**

(21) Anmeldenummer: **81110067.6**

(22) Anmeldetag: **02.12.81**

(54) **Verfahren zum Herstellen von Keten.**

(30) Priorität: **08.12.80 DE 3046219**

(43) Veröffentlichungstag der Anmeldung:
**16.06.82 Patentblatt 82/24**

(45) Bekanntmachung des Hinweises auf die Patenterteilung:
**16.05.84 Patentblatt 84/20**

(84) Benannte Vertragsstaaten:
**BE CH DE FR GB IT LI NL**

(56) Entgegenhaltungen:
**DE - B - 1 643 758**
**FR - A - 1 091 934**
**US - A - 2 108 829**

(73) Patentinhaber: **HOECHST AKTIENGESELLSCHAFT,
Postfach 80 03 20, D-6230 Frankfurt am Main 80 (DE)**

(72) Erfinder: **Arnold, Dieter, Ölmühlweg 8a,
D-6240 Königstein/Taunus (DE)**
Erfinder: **Bartels, Jörg, Am Honigbaum 2c,
D-6239 Eppstein/Taunus (DE)**
Erfinder: **Lenzmann, Heinrich, Brunhildenweg, 22,
D-6233 Kelkheim (Taunus) (DE)**
Erfinder: **Jacobsen, Günter, Dr., Goerdelerstrasse 8,
D-6050 Offenbach/Main (DE)**
Erfinder: **Wendt, Heinz, Dr., Kronberger Weg 20,
D-6231 Sulzbach (DE)**
Erfinder: **Stoltenberg, Manfred, Wachtelweg 10,
D-6230 Frankfurt am Main 80 (DE)**

**0 053 793**

## Verfahren zum Herstellen von Keten

Die Erfindung bezieht sich auf ein Verfahren zum Herstellen von Keten durch thermische, katalytische Spaltung von Essigsäure unter vermindertem Druck und durch Abkühlen der heißen Spaltgase auf ca. 0°C bis −10°C wobei Wasser, nicht umgesetzte Essigsäure und Essigsäureanhydrid kondensiert werden.

Bei einem bekannten technischen Verfahren zum Herstellen von Keten wird Essigsäure kontinuierlich in Gegenwart von beispielsweise Triäthylphosphat als Katalysator oberhalb 700°C und in einem Druckbereich von etwa 100 bis 500 mbar hergestellt. Dem heißen Spaltgas setzt man kurz nach Verlassen der Reaktionszone Ammoniak hinzu, um Phosphorsäure zu binden. Das Spaltgas enthält außer Keten und Wasser die folgenden aus Nebenreaktionen stammenden Gase: Methan, Kohlendioxid, Kohlenmonoxid, Äthylen, Allen und in geringen Mengen höhere Kohlenwasserstoffe sowie ungespaltene Essigsäure und Ammoniumsalze der Essigsäure und der Phosphorsäure.

Zur Gewinnung des Ketens aus dem Spaltgas wird dieses zunächst möglichst rasch abgekühlt, um die kondensierbaren Anteile abzutrennen. Dabei läßt es sich nicht vermeiden, daß Keten mit Wasser und Essigsäure in Berührung kommt und unter Bildung von Essigsäure und Essigsäureanhydrid reagiert. Man erhält also bei der Abkühlung ein Kondensat, bestehend aus Wasser, Essigsäure und Essigsäureanhydrid. Die unerwünschte Folge dieser Reaktion, bei der ein Teil des entstandenen Ketens wieder in Ausgangsprodukt verwandelt wird, ist eine Verringerung sowohl des Essigsäure-Umsatzgrades als auch eine Erhöhung des Energiebedarfs der Anlage. Die Kondensation im Vakuum ist auch bei tiefen Temperaturen unvollständig. Das Spaltgas enthält noch schwer abzutrennende Nebel von Essigsäure und Essigsäureanhydrid.

Nach dem aus der deutschen Auslegeschrift 1 250 811 bekannten Verfahren wird das ca. 700°C heiße Spaltgas zunächst auf 50°C abgekühlt, dann über einen Zyklonabscheider geführt und weiter gekühlt. Vorhandener Nebel wird nach diesem Verfahren nicht entfernt. Unter diesen Bedingungen ist die Rückreaktion insbesondere bei Anlagen mit größeren Durchsätzen beträchtlich, erkennbar an der hohen Konzentration der Essigsäure im Kondensat.

Nach der deutschen Patentschrift 1 643 759 ist es bekannt, die Spaltgase zunächst auf ca. 120°C vorzukühlen, um sie dann durch eine perforierte Kühlstrecke zu leiten in der das Kondensat sofort abfließen kann, wobei die Kühlstrecke mit Kühlsole auf einer Temperatur von −20 bis −30°C gehalten wird. Anschließend wird das Rohketen gegen Prallwände geleitet um die Essigsäure- und Essigsäureanhydridnebel zu entfernen. Die Prallwände werden auf einer Temperatur zwischen −30° und −50°C gehalten. Damit die Temperatur der Prallwände niedrig gehalten werden kann, ist zwischen perforierter Kühlstrecke und den Prallwänden eine Verweilstrecke vorgesehen, die auf einer Temperatur zwischen 0 und +10°C gehalten wird. In dieser Verweilstrecke reagiert die noch vorhandene Essigsäure mit dem Rohketen. Es entsteht Essigsäureanhydrid, welches als solches abgeschieden wird. Der Gasstrom wird bei einer Geschwindigkeit von 200 bis 2 m/sec und einem Druck von 50 bis 500 mbar gehalten. Das austretende Keten ist weitgehend frei von Nebel enthält aber noch Reste an kondensierbaren Verunreinigungen. Nachteile dieses Verfahrens sind ein aufwendiges, schwer zu reinigendes Kondensationssystem sowie die Erfordernisse sehr tiefer Temperaturen und somit hoher Energiebedarf.

Aufgabe zu vorliegender Erfindung ist demnach ein Verfahren zum Herstellen von Keten zu schaffen, nach dem eine möglichst hohe Ketenausbeute bezogen auf die eingesetzte Essigsäure bei niedrigem Energiebedarf und unter Verwendung einfacher Kondensatoren möglich ist. Die Aufgabe wird durch ein Verfahren zum Herstellen von Keten gelöst, das dadurch gekennzeichnet ist, daß das Spaltgas durch einen oder mehrere hintereinander geschaltete Rohrbündelwärmetauscher mit einem Volumen V$[m^3]$, Druckverlusten von 50 bis 150 mbar und einem Oberflächen-Volumenverhältnis von 60 bis 120 $m^{-1}$ geleitet und das Kondensat abgezogen wird, wobei die Menge der zur thermischen Spaltung eingesetzten Essigsäure 0,5 bis 2,5 t · $h^{-1}$ · $m^{-3}$, bezogen auf das Volumen V des Rohrbündelwärmetauschers, beträgt.

Wie bereits erwähnt, enthält das Spaltgas auch feste Anteile, wie verkohltes Material und gegebenenfalls Ammoniumsalze, die sich im Kondensationssystem niederschlagen, wodurch der Druck im Spaltofen ansteigt und die Reaktion sich verschlechtert. In dem Bestreben, eine möglichst lange Laufzeit der Anlage zu erzielen, hat man deshalb die Kondensatoren bisher großzügig dimensioniert. Es war überraschend und nicht vorhersehbar, daß durch Verkleinerung des Volumens des gesamten Kondensationssystems mit einhergehender beträchtlicher Erhöhung der Gasgeschwindigkeit die Rückreaktion in hohem Maße herabgesetzt und der Essigsäureumsatzgrad erhöht werden konnte, ohne daß sich das Kondensatorsystem vorzeitig zusetzt. Die Steigerung des Druckverlustes hat dabei keinen schädlichen Einfluß auf den Umsatz. Bei dem erfindungsgemäßen Verfahren können in der Technik übliche genormte Apparate verwendet werden; komplizierte Sonderkonstruktionen sind nicht erforderlich.

## Beispiel

3,6 t/h Essigsäure werden verdampft und die Dämpfe in das Spaltrohrsystem eines mit Erdgas beheizten Ketenofens geleitet. Nach Vorheizen wird dem Essigsäuredampf als Katalysator Triäthylphosphat hinzugesetzt. In der sich anschließenden Spaltzone wird die Temperatur bis auf 700°C gesteigert. Am Ofenausgang herrscht ein Druck von 358 mbar. Dort wird dem heißen Gasgemisch (Spaltgas) Ammoniak hinzugefügt.

Das Gemisch wird nun auf dem kürzesten Weg rohrseitig durch eine Reihe von 5 Röhrenwärmetauschern geleitet. Die ersten zwei Wärmetauscher werden mit Kühlwasser, die restlichen drei mit Kühlsole von −10°C beaufschlagt. Die 5 Wärmetauscher sind unter sich gleich, je 2500 mm lang, ihr Durchmesser 700 mm, ihre Anzahl der Rohre 290 mit einer lichten Weite von 25 mm und einer gesamten inneren Oberfläche von 285 m². Das gesamte Volumen der Kondensatoren einschließlich Hauben und Abscheider beträgt 3,5 m³, das Verhältnis Oberfläche zu Volumen 81,5 m$^{-1}$.

Die Reihe der Wärmetauscher besitzt eine Neigung gegen die Waagrechte von 5°. Das gebildete Kondensat wird in der hinteren Haube jedes Wärmetaschers zusammengeführt und in einen Sammelbehälter abgelassen. Zwischen dem dritten und vierten Rohrbündel ist ein Prallabscheider angeordnet. Das Rohketen verläßt den fünften Wärmetauscher mit einer Temperatur von −5°C und einem Druck von 266 mbar. Der Druckverlust des Kondensationssystems beträgt 358 mbar − 266 mbar = 92 mbar.

Die Gasgeschwindigkeit beträgt 30−80 m/sec.

Es werden erhalten

1,94 t/h Keten und 1,46 t/h einer 38%igen wäßrigen Essigsäure. Das entspricht einem Umsatz der eingesetzten Essigsäure von 84%, einer Ketenausbeute von 92%, bezogen auf die umgesetzte Essigsäure und einer Ketenausbeute von 77%, bezogen auf die eingesetzte Essigsäure.

## Vergleichsbeispiel

Die Spaltung der Essigsäure erfolgt wie im Beispiel angegeben mit den dort angeführten Mengen. Der Druck am Ofenausgang beträgt 175 mbar.

Das Kondensationssystem besteht aus einer Reihe von 4 Wärmetauschern, ebenfalls mit rohrseitiger Gasführung. Die ersten zwei Wärmetauscher werden mit Kühlwasser, die restlichen zwei mit Kühlsole von −10°C beaufschlagt. Die Abmessungen der verschiedenen großen Rohrbündel sind aus folgender Tabelle ersichtlich.

| Wärmetauscher Nr. | Länge mm | Durchmesser mm | Anzahl der Rohre | lichte Weite der Rohre | innere Oberfläche m² | einschl. Hauben Volumen m³ | Verhältnis Oberfläche /Volumen m$^{-1}$ |
|---|---|---|---|---|---|---|---|
| 1 | 812 | 1600 | 1247 | 25 | 79 | 2,9 | 27,3 |
| 2 | 1880 | 1750 | 1492 | 25 | 220 | 3,35 | 65,6 |
| 3 | 2500 | 1350 | 794 | 25 | 155 | 3,24 | 47,8 |
| 4 | 2500 | 800 | 353 | 21 | 58 | 0,96 | 60,4 |
| Summe | | | | | 512 | 10,45 | 48,97 |

Die Reihe der Wärmetauscher besitzt eine Neigung gegen die Waagrechte von 27°.

Das gebildete Kondensat wird, wie im Beispiel angegeben, in den Hauben gesammelt und in einen Sammelbehälter abgelassen. Das Rohketen verläßt den vierten Wärmetauscher mit einer Temperatur von −5°C und einem Druck von 162 mbar. Der Druckverlust des Kondensationssystems beträgt als 175 mbar − 162 mbar = 13 mbar. Die Gasgeschwindigkeit beträgt 15 bis 25 m/sec.

Es werden erhalten 1,35 t/h Keten und 2,11 t/h einer 70%igen wäßrigen Essigsäure. Das entspricht einem Umsatz der eingesetzten Essigsäure von 58%, einer Ketenausbeute von 92%, bezogen auf die umgesetzte Essigsäure und einer Ketenausbeute von 54%, bezogen auf die eingesetzte Essigsäure.

## Patentanspruch

Verfahren zum Herstellen von Keten durch thermische, katalytische Spaltung von Essigsäure unter vermindertem Druck und durch Abkühlen der heißen Spaltgase auf ca. 0 bis −10°C, wobei Wasser, nicht umgesetzte Essigsäure und Essigsäureanhydrid kondensiert werden, dadurch gekennzeichnet,

**0 053 793**

daß das Spaltgas durch einen oder mehrere hintereinander geschaltete Rohrbündelwärmetauscher mit einem Volumen V[m$^3$], Druckverlusten von 50 bis 150 mbar und einem Oberflächen-Volumenverhältnis von 60 bis 120 m$^{-1}$ geleitet und das Kondensat abgezogen wird, wobei die Menge der zur thermischen Spaltung eingesetzten Essigsäure 0,5 bis 2,5 t · h$^{-1}$ · m$^{-3}$, bezogen auf das Volumen V des Rohrbündelwärmetauschers beträgt.

## Claim

A process for the preparation of ketene by thermal, catalytic cracking of acetec acid under reduced pressure and by cooling the hot cracked gases to approx. 0 to −10°C, water, unreacted acetic aced and acetic anhydride being condensed, which comprises passing the cracked gas through one or more shell-and-tube heat exchangers arranged in tandem and having a volume V(m$^3$), a pressure drop of 50 to 150 mbars and a surface/volume ratio of 60 to 120 m$^{-1}$, and drawing off the condensate, it being necessary for the quantity of acetic aced employed for the thermal cracking to be 0.5 to 2.5 t · hours$^{-1}$ · m$^{-3}$, relative to the volume V of the shell-and-tube heat exchanger.

## Revendication

Procédé de préparation du cétène par craquage catalytique à chaud de l'acide acétique, sous pression réduite, et refroidissement des gaz de craquage chauds à une température d'environ 0 à −10°C, l'eau, l'acide acétique qui n'a pas réagi et l'anhydride acétique étant condensés, procédé caractérisé en ce qu'on fait passer le gaz de craquage par un échangeur de chaleur à faisceau tubulaire, ou par plusieurs échangeurs de ce genre montés en série, présentant un volume V(m$^3$), des pertes de pression de 50 à 150 mbar et un rapport de la surface au volume compris entre 60 et 120 m$^{-1}$, et on soutire le condensat, la quantité de l'acide acétique mis en jeu dans le craquage thermique étant comprise entre 0,5 et 2,5 t · h$^{-1}$ · m$^{-3}$, relativement au volume V de l'échangeur de chaleur à faisceau tubulaire.